Europäisches Patentamt

European Patent Office

Office européen des brevets

(11) Veröffentlichungsnummer: **0 361 475**

**A1**

(12) # EUROPÄISCHE PATENTANMELDUNG

(21) Anmeldenummer: **89117958.2**

(22) Anmeldetag: **28.09.89**

(51) Int. Cl.5: **C07K 3/08 , C07K 3/20 , C12N 15/58 , A61K 37/54**

Der (Die) Mikroorganismus (Mikroorganismen) ist (sind) bei der Deutsche Sammlung von Mikroorganismen unter der (den) Nummer(n) DSM 3689 hinterlegt worden.

(30) Priorität: **28.09.88 DE 3832898**

(43) Veröffentlichungstag der Anmeldung: **04.04.90 Patentblatt 90/14**

(84) Benannte Vertragsstaaten: **AT BE CH DE ES FR GB GR IT LI LU NL SE**

(71) Anmelder: **BOEHRINGER MANNHEIM GMBH Sandhofer Strasse 112-132 D-6800 Mannheim-Waldhof(DE)**

(72) Erfinder: **Rudolph, Rainer Färbergasse 19 D-8120 Weilheim(DE)**
Erfinder: **Opitz, Ulrich Anton-Roth-Strasse 2 D-8170 Bad Tölz(DE)**
Erfinder: **Kohnert, Ulrich Heubachweg 6 D-8121 Habach(DE)**
Erfinder: **Fischer, Stephan Jakobifeldweg 11 D-8121 Polling(DE)**

(74) Vertreter: **Huber, Bernhard, Dipl.-Chem. et al Möhlstrasse 22 Postfach 860 820 D-8000 München 86(DE)**

(54) **Präparat von in Prokaryonten exprimiertem Plasminogenaktivator.**

(57) Ein Präparat von rekombinantem, in Prokaryonten exprimiertem p-t-PA weist einen Gehalt an aktivem p-t-PA von mehr als 90% und eine spezifische Aktivität von mehr als $0,4 \times 10^6$ IU/mg auf. Zur Herstellung eines solchen Präparats von rekombinantem, in Prokaryonten exprimiertem p-t-PA durch Aufschluß der exprimierenden Prokaryontenzellen, Solubilisierung unter denaturierenden und reduzierenden Bedingungen und Reaktivierung unter oxidierenden Bedingungen konzentriert man im Reaktivierungsansatz und führt dann eine Reinigung mittels Affinitätschromatographie durch. Ein erfindungsgemäßes p-t-PA-Präparat kann zur Auflösung von Blutgerinnseln verwendet werden.

EP 0 361 475 A1

## Präparat von in Prokaryonten exprimiertem Plasminogenaktivator

Die Erfindung betrifft ein Präparat von rekombinantem, in Prokaryonten exprimiertem Plasminogenaktivator (p-t-PA), das einen besonders hohen Gehalt an aktivem p-t-PA und eine besonders hohe biologische Aktivität aufweist, ein Verfahren zur Herstellung des Präparats, sowie seine Verwendung.

Die Aktivierung des fibrinolytischen Systems verläuft unter physiologischen Bedingungen in einer ähnlichen Reaktionskaskade wie die Blutgerinnung. Die zentrale Reaktion dieser Kaskade ist die Aktivierung von Plasminogen zu Plasmin. Plasmin wiederum löst Fibrin, die Hauptkomponente der Proteinmatrix von geronnenem Blut. Aufgrund seiner Fähigkeit, spezifisch an Fibrin zu binden, wird der Gewebs-Plasminogen-Aktivator t-PA (tissue type plasminogen activator) im allgemeinen als der physiologisch wirksame Plasminogen-Aktivator angesehen (Matsuo et al., Nature 291 (1981), 590). Diese auf die Fibrinoberfläche fokusierte Aktivität des Enzyms ließ es als geeignetes Mittel zur Bekämpfung von pathologischen Aderverschlüssen (z. B. beim Herzinfarkt) erscheinen. In umfangreichen klinischen Versuchen konnten die in die Anwendung von t-PA gesetzten Hoffnungen größtenteils bestätigt werden (Collen et al., Circulation 70, (1984), 1012; Circulation 73, (1986), 511).

Es wurden daher bereits einige Methoden zur Gewinnung von t-PA in großen Mengen vorgeschlagen. So wurden z. B. verschiedene Säuger-Zellinien beschrieben, welche die für den therapeutischen Einsatz benötigten t-PA-Mengen produzieren können (Riiken und Collen, J. Biol. Chem. 256, (1981), 7035; Collen et al., J. Pharm. Exp. Ther. 231, (1984), 146; Kaufman et al., Mol. (Cell Biol. 5 (1985), 1750). Die Kultur derartiger Zellen war aber relativ aufwendig und teuer. Seit der Klonierung der t-PA cDNA in E. coli (Pennica et al., Nature 30, (1983), 214-221) stand auch der Weg zur Expression des Enzyms in einem prokaryontischen System offen. In Prokaryonten hergestellter Plasminogenaktivator ist im Gegensatz zum t-PA aus Säugerzellen, der an 2 bis 3 Stellen glykosyliert ist, unglykosyliert. Es wird daher in folgenden zur Unterscheidung von t-PA aus Eukaryonten p-t-PA (prokaryontischer t-PA) benannt. p-t-PA fällt jedoch in den Bakterien, wie viele andere in Bakterien exprimierte eukaryontische Proteine, in inaktiver aggregierter Form, den sogenannten "inclusion bodies" (IB's), an und es findet keine korrekte Faltung zur nativen aktiven Struktur statt. Häufig sind die Protein-Aggregate dabei noch durch bakterielle Proteine verunreinigt. Im Fall von p-t-PA sind dies hauptsächlich "outer membrane"-Proteine und der Elongationsfaktor EF-Tu (Brinkmann, 1987, Diplomarbeit, Universität München). Derartige verunreinigte Proteinpräparationen, die zusätzlich noch in Form der unlöslichen inclusion bodies anfallen, müssen, um p-t-PA in seiner aktiven Form zu erhalten, nach der Expression in den Prokaryontenzellen zuerst solubilisiert, denaturiert und dann, um die richtige Faltung der Proteine zu bewirken, renaturiert werden. Jedoch konnte durch die bisher bekannten Verfahren zur Reaktivierung nur ein relativ kleiner Anteil des in den inclusion bodies vorliegenden Materials in aktiver Form erhalten werden, so daß nur eine verhältnismäßig geringe spezifische Aktivität von p-t-PA nach der Renaturierung erhalten werden konnte. Zusätzlich war der Gehalt an aktivem p-t-PA durch das Vorhandensein von Fremdproteinen beeinträchtigt.

Aufgabe der vorliegenden Erfindung ist es daher, p-t-PA, welche in Prokaryonten exprimiert wurde, in möglichst hoher Ausbeute und möglichst hoher Reinheit bereitzustellen.

Gelöst wird die Aufgabe erfindungsgemäß durch ein Präparat von rekombinantem, in Prokaryonten exprimiertem p-t-PA, das einen Gehalt an aktivem p-t-PA von mehr als 90%, bezogen auf Gesamtprotein, und eine spezifische Aktivität von mehr als $0,4\times10^6$ IU/mg aufweist.

In einer bevorzugten Ausführungsform weist das erfindungsgemäße p-t-PA-Präparat einen Gehalt an aktivem p-t-PA von mehr als 95%, insbesondere von mehr als 98% auf.

Ein weiterer Gegenstand der Erfindung ist ein Verfahren zur Herstellung eines Präparats von rekombinantem, in Prokaryonten exprimiertem p-t-PA mit einem Gehalt an aktivem p-t-PA von mehr als 90 %, bezogen auf Gesamtprotein, und einer Aktivität von mindestens $0,4\times10^6$ IU/mg, durch Aufschluß der exprimierenden Prokaryontenzellen, Isolierung der IB's, Solubilisierung der IB's unter denaturierenden und reduzierenden Bedingungen, Derivatisierung zu gemischten Disulfiden und Reaktivierung unter kontrollierten Redox-Bedingungen, welches dadurch gekennzeichnet ist, daß man nach der Reaktivierung p-t-PA im Reaktivierungsansatz konzentriert und daran anschließend eine chromatographische Reinigung mittels Affinitätschromatographie durchführt.

Es wurde festgestellt, daß es durch das erfindungsgemäße Verfahren überraschenderweise gelingt, in Prokaryonten exprimierten p-t-PA besonders rein und frei von Fremdproteinen und mit einem Gehalt an aktivem p-t-PA von mehr als 90% herzustellen.

Für das Verfahren sind alle Prokaryonten geeignet, die t-PA-Plasmide enthalten. Derartige Prokaryonten und Plasmide sind dem Fachmann bekannt. Besonders geeignet sind die in EP-A-0 242 835 beschriebenen Plasmide, wobei das Plasmid pePa 133 bevorzugt eingesetzt wird.

Der Zellaufschluß der exprimierenden Prokaryonten wird in an sich bekannter Weise durchgeführt, beispielsweise durch Behandlung mit Lysozym in Gegenwart von Triton-X-100 und NaCl und anschließender Hochdruckdispersion.

Die Solubilisierung unter denaturierenden oder reduzierenden Bedingungen, sowie die Reaktivierung unter oxidierenden Bedingungen können nach an sich bekannten Methoden, wie sie beispielsweise in EP-A-0 253 823 beschrieben sind, durchgeführt werden.

In der Stufe der Solubilisierung können als Denaturierungsmittel alle üblicherweise verwendeten Denaturierungsmittel oder aber auch Arginin eingesetzt werden. Bevorzugt sind die bekannten Denaturierungsmittel Guanidin-Hydrochlorid oder Harnstoff oder dessen Derivate. Ferner können Gemische dieser Denaturierungsmittel verwendet werden. Weiter können Denaturierungsmittel verwendet werden, wie sie z. B. aus der EP-A-0 114 506 bekannt sind. Als Reduktionsmittel werden bevorzugt Reduktionsmittel aus der Mercaptangruppe wie z. B. reduziertes Glutathion (GSH) oder 2-Mercaptoethanol verwendet. Dies kann beispielsweise in einer Konzentration von ca. 50 bis 400 mmol/l geschehen. Weiter sind bevorzugte Reduktionsmittel DTE (Dithioerythritol) bzw. DTT (Dithiothreitol), z. B. in einer Konzentration von ca. 80 bis 400 mmol/l. Die Solubilisierung erfolgt zweckmäßigerweise bei Raumtemperatur während einer Dauer von 1 bis mehreren Stunden, vorzugsweise von 2 Stunden. Zur Verhinderung der Aufoxidation des Reduktionsmittels durch Luftsauerstoff kann es auch zweckmäßig sein, EDTA zuzusetzen.

Die Renaturierung kann, wie in der obengenannten europäischen Anmeldung EP-A-0 253 823 beschrieben, direkt, oder über eine Überführung des p-t-PA in das gemischte Disulfid von p-t-PA und GSSG bewirkt werden. Die hierfür nötigen Reaktionsbedingungen sind ebenfalls in der europäischen Anmeldung näher ausgeführt.

Erfindungsgemäß wird beim Verfahren zur Herstellung des p-t-PA-Präparats nach der Reaktivierung p-t-PA im Reaktivierungsansatz konzentriert und darauf folgend eine Abtrennung von Fremdprotein über eine Affinitätschromatographie bewirkt.

Die Konzentration von p-t-PA im Reaktivierungsansatz kann nach an sich bekannten Methoden durchgeführt werden, jedoch ist insbesondere für kleinere Volumina die Durchführung einer Ammoniumsulfatfällung bevorzugt, wogegen bei größeren Volumina eine Konzentration in einem Hämodialysator bevorzugt wird.

Im Falle der Durchführung einer Ammoniumsulfatfällung wird das Pellet nach der Zentrifugation zweckmäßigerweise nochmals gewaschen, und nach erneutem Zentrifugieren in Reoxidationspuffer gelöst.

Das erfindungsgemäße Verfahren wird bevorzugt in Anwesenheit von L-Arginin, insbesondere in einer Konzentration von 25 bis 1000 mmol/l, durchgeführt.

Die erfindungsgemäße Abtrennung von Fremdprotein über eine Affinitätschromatographie wird in einer bevorzugten Ausführungsform der Erfindung über eine ETI (Erythrina-Trypsin-Inhibitor)-Adsorbersäule durchgeführt. Hierbei ist ETI auf einem Trägermaterial (Adsorber), wie z.B. Sepharose fixiert. Die Reinigung über eine ETI-Adsorbersäule hat den Vorteil, daß das ETI-Adsorber-Säulenmaterial direkt aus dem konzentrierten Reoxidationsansatz sogar in Gegenwart von so hohen Argininkonzentrationen wie 0,8 mmol/l Arginin beladen werden kann. Eine Aggregation von p-t-PA, wie sie bei niedrigen Argininkonzentrationen unter 25 mmol/l stattfinden kann, wird dadurch vermieden. Besonders bevorzugt erfolgt daher die Reinigung des p-t-PA-Präparates über eine ETI-Absorbersäule in Gegenwart von 0,5 bis 1,0 M Arginin. Die p-t-PA enthaltende Lösung hat dabei bevorzugt einen pH von mehr als 6,5, besonders bevorzugt mehr als 7.

Die Elution von der ETI-Säule erfolgt durch pH-Erniedrigung, sowohl in Gegenwart als auch in Abwesenheit von Arginin unter Bedingungen, die eine gute Löslichkeit von t-PA, das in Prokaryonten exprimiert wurde, ermöglichen. Der pH liegt bei der bevorzugt Elution im schwach sauren Bereich, besonders bevorzugt im Bereich von 5,5 bis 6,5.

Ein erfindungsgemäß hergestelltes p-t-PA-Präparat besitzt eine spezifische t-PA-Aktivität von $\geq$ 0,4 $\cdot 10^6$ IU/mg, dessen Stimulierbarkeit durch Fibrinogenspaltprodukte (Aktivität in Gegenwart von Fibrinogenpeptiden/Aktivität ohne Fibrinogenpeptide) größer als 10fach ist. Die Reinheit des erfindungsgemäßen Präparats, die mehr als 90% und bevorzugt mehr als 95%, insbesondere mehr als 98% beträgt, wurde durch SDS-PAGE und RP-HPLC (Umkehrphasen-HPLC) nachgewiesen. Die Fähigkeit zur Blutpfropfenlyse des erfindungsgemäß hergestellten p-t-PA-Präparats wurde im Kaninchenmodell nachgewiesen. Weiterhin wurde die Abbaurate des erfindungsgemäßen p-t-PA-Präparats in Kaninchenmodellen gemessen. Verglichen mit in Eukaryonten exprimiertem t-PA verschwindet das erfindungsgemäße p-t-PA-Präparat deutlich langsamer aus dem Plasma.

Ein weiterer Gegenstand der Erfindung ist daher die Verwendung des erfindungsgemäßen p-t-PA-Präparats mit hoher Reinheit und mit hohem Gehalt an aktivem p-t-PA in einem Mittel zur Auflösung von Blutgerinnseln bei Mensch und Tier. Ein derartiges erfindungsgemäßes Mittel kann weitere pharmazeutisch akzeptierbare Zusatzstoffe und Hilfsstoffe, wie z.B. Arginin, Harnstoff (z.B. jeweils 100 mmol/l) und Pufferkomponenten enthalten.

Die folgenden Beispiele erläutern in Verbindung mit den Abbildungen die Erfindung weiter.

Fig. 1 zeigt eine SDS-PAGE vor und nach Reinigung mittels Affinitätschromatographie über ETI-Sepharose;

Fig. 2 zeigt eine RP-HPLC nach Reinigung von p-t-PA aus E. coli mittels Affinitätschromatographie über ETI-Sepharose.

Fig. 3 zeigt die Bindung in vitro von p-t-PA aus E. coli an Fibrin und

Fig. 4 zeigt den Verlauf des Aktivitätsspiegels von p-t-PA während und nach der Infusion in Kaninchen.

**Beispiel 1**

Präparation von aktivem p-t-PA aus E. coli

Zellyse und Präparation der inclusion bodies (IB's)

1,6 kg Zellfeuchtmasse (E. coli, DSM 3689, transformiert mit dem in EP-A-0 242 835 beschriebenen Plasmid pePa 133) wurden in 10 l 0,1 mol/l Tris- HCl, 20 mmol/l EDTA, pH 6,5, 4°C suspendiert. Dazu wurde 2,5 g Lysozym zugegeben und 30

Minuten bei 4°C inkubiert; anschließend wurde vollständiger Zellaufschluß mittels Hochdruckdispersion durchgeführt. Zur Aufschlußlösung wurden 5 l 0,1 mol/l Tris-HCl, 20 mmol/l EDTA, 6 % Triton-X-100 und 1,5 mol/l NaCl, pH 6,5 zugemischt und weitere 30 Minuten bei 4°C inkubiert. Daran anschließend folgte die Abtrennung der unlöslichen Bestandteile (IB's) durch Zentrifugation.

Das Pellet wurde in 10 l 0,1 mol/l Tris-HCl, 20 mmol/l EDTA, pH 6,5 suspendiert, 30 Minuten bei 4°C inkubiert und IB-Präparat durch anschließende Zentrifugation isoliert.

Solubilisierung der IB's

100 g IB's (Naßgewicht) wurden in 450 ml 0,1 mol/l Tris-HCl, 6 mol/l Guanidinium HCl, 0,2 mol/l DTE, 1 mmol/l EDTA, pH 8,6 suspendiert und 2,5 Stunden bei 25°C gerührt.

Nach Einstellen des pH-Wertes auf pH 3 mit HCl (25%) wurde eine Dialyse gegen 10 mmol/l HCl (3 x 50 l, 24 Stunden, 4°C) durchgeführt.

Derivatisierung

Guanidinium HCl (fest) wurde vorgelegt, so daß nach Endverdünnung des obengenannten Dialysats mit 10 mmol/l HCl die Guanidinium HCl-Konzentration 6 mol/l betrug.

Der Ansatz wurde bei 25°C 1,5 Stunden vorinkubiert, anschließend wurde GSSG ad 0,1 mol/l und Tris-HCl ad 0,05 mol/l eingestellt und der pH-Wert mit 5 mol/l NaOH auf pH 9,3 titriert. Der Ansatz wurde 3,5 Stunden bei 25°C gerührt.

Nach Einstellung des pH-Wertes auf pH 3 mit HCl (25%) wurde eine Dialyse gegen 10 mmol/l HCl (3 x 100 l, 48 Stunden, 4°C) durchgeführt. Nach der Dialyse wurde zentrifugiert und der klare Überstand weiterverarbeitet.

Naturierung

Ein 10 l Reaktionsgefäß wurde mit 0,1 mol/l Tris-HCl, 0,8 mol/l L-Arginin, 2 mmol/l GSH, 1 mmol/l EDTA, pH 8,5 gefüllt. Die Renaturierung wurde bei 20°C durch dreifache Zugabe von jeweils 100 ml Derivat (gemischtes Disulfid, s. o.) im Zeitabstand von 24 Stunden bewirkt.

Nach der Renaturierung erhielt man ein Präparat mit einer spezifischen Aktivität von 10.000 bis 20.000 IU/mg (Test nach J.H.Verheijen et al. (Thromb. Haemostas. 48, 266, 1982), modifiziert nach H. Lill (2. Ges. Inn. Med. 42, 478, 1987)).

Konzentrierung

Die Renaturierungslösung kann, bei Bedarf, durch Fällung mit Ammoniumsulfat bzw. Ultrafiltration aufkonzentriert werden.

Bei der Ammoniumsulfat-Fällung wurde der pH-Wert der Renaturierungslösung auf pH 7,5 eingestellt. Die Fällung erfolgte durch Zugabe von festem $(NH_4)_2SO_4$ ad 60 % bei 4° C. Nach einer Stunde Rühren bei 0° C wurde zentrifugiert und die Resolubilisierung des Pellets in 1/10 bis 1/100 des Ausgangsvolumens mit 0,8 mol/l L-Arginin, 2 mmol/l GSH, 1 mmol/l EDTA, pH 8,5 durchgeführt.

**Beispiel 2**

Reinigung von p-t-PA aus E. coli durch Affinitätschromatographie mittels Erythrina Trypsin Inhibitor (ETI)

Präparation des Affinitätsadsorbers

ETI wurde nach Vorschrift des Herstellers an BrCN-aktivierte Sepharose von Pharmacia gekoppelt.

Affinitätschromatographie ohne vorherige Konzentrierung des Renaturierungsansatzes

Eine ETI-Sepharosesäule (V = 5 ml) wurde mit 0,1 mol/l Tris-HCl, 0,8 mol/l L-Arginin, 2 mmol/l GSH, 1 mmol/l EDTA, pH 8,5 äquilibriert.

Dann wurde 1 l Renaturierungslösung (Beispiel 1) mit einer Flußrate von 10 Säulenvolumen/Stunde aufgetragen. Mit Äquilibrierungspuffer wurde nachgewaschen, bis die Adsorption bei 280 nm den Leerwert des Puffers erreichte.

Die Elution von p-t-PA erfolgte durch pH-Sprung mit 0,1 mol/l Tris-HCl, 0,8 mol/l L-Arginin, 2 mmol/l GSH, 1 mmol/l EDTA, pH 5.

|  | Volumen | Aktivität | c | $A_{sp}$ spezifische Aktivität |
|---|---|---|---|---|
|  | (ml) | (IU/ml) | (mg/ml) | (IU/mg) |
| Reoxidationslösung | 1000 | 3480 | 0,15 | 23200 |
| p-t-PA-Pool | 5 | 682500 | 1,22 | 560000 |

Affinitätschromatographie nach Konzentrierung der Renaturierungslösung mittels Ultrafiltration

Die Renaturierungslösung wurde mittels Haemodialysator 1:45 eingeengt. Das Konzentrat wurde mit NaCl auf eine Konzentration von 0,5 mol/l aufgestockt.

Eine ETI-Sepharose-Säule (V = 80 ml) wurde mit 0,1 mol/l Tris-HCl, 0,8 mol/l L-Arginin, 0,5 mol/l NaCl, pH 7,5 äquilibriert. 700 ml Konzentrat wurden mit einer Flußrate von 4 Säulenvolumen/Stunde aufgetragen. Danach wurde mit Äquilibrationspuffer nachgewaschen, bis die Absorption bei 280 nm den Leerwert des Puffers erreichte.

Die Elution von p-t-PA erfolgte mit 20 mmol/l Zitronensäure, pH 3,2.

|  | Volumen | Aktivität | c | $A_{sp}$ spezifische Aktivität |
|---|---|---|---|---|
|  | (ml) | (IU/ml) | (mg/ml) | (IU/mg) |
| Konzentrat | 700 | 247000 | 25 | 10000 |
| p-t-PA-Pool | 1000 | 155000 | 0,18 | 874000 |

**Beispiel 3**

Charakterisierung von gereinigtem p-t-PA aus E. coli

Proteinchemische Charakterisierung

- SDS-PAGE und RP-HPLC (Umkehrphasen-(reverse-phase)-HPLC)

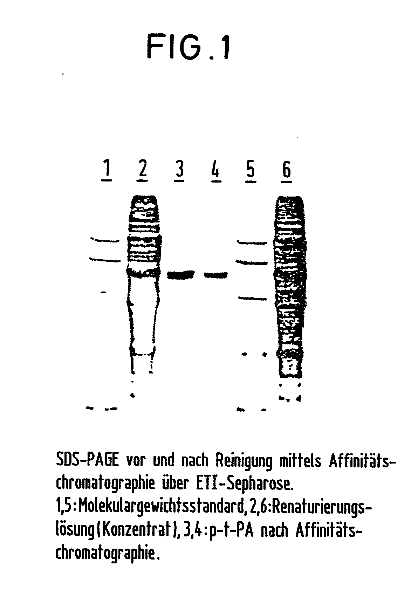

Die Homogenität des durch Affinitätschromatographie über ETI-Sepharose gereinigten Präparats wurde mittels SDS-Elektrophorese und RP-HPLC überprüft. Fig. 1 zeigt im Vergleich die elektrophoretische Analyse von konzentrierter Renaturierungslösung und gereinigtem Protein. Eine densitometrische Analyse des Gels zeigt, daß man durch Affinitätschromatographie über ETI-Sepharose eine Anreicherung von p-t-PA auf > 95% erzielt. Aus den relativen Laufstrecken kann für p-t-PA aus E. coli ein Molekulargewicht von 60 ± 2 kDa abgeschätzt werden.

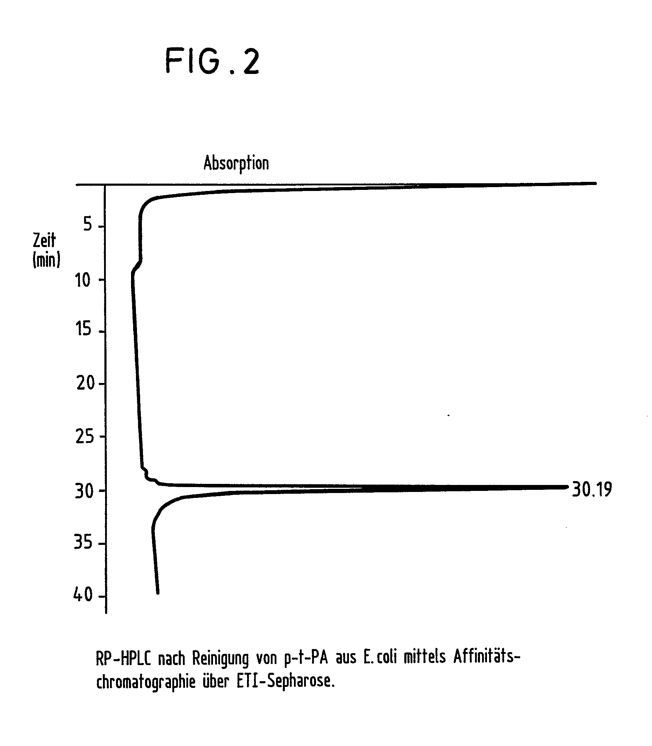

Proteine unterscheiden sich in ihren hydrophoben Wechselwirkungen mit einer polymeren hydrophoben Matrix. Diese charakteristische Eigenschaft wird bei der Reverse Phase HPLC als analytische Methode zur Reinheitskontrolle verwendet. Die Analyse des gereinigten p-t-PA's erfolgte über eine Nucleosil 300 (Knauer) Trennsäule mittels Trifluoressigsäure/Acetonitril Gradient (Puffer A: 1,2 ml Trifluoressigsäure in 1000 ml $H_2O$; Puffer B: 300 ml $H_2O$, 700 ml Acetonitril, 1 ml Trifluoressigsäure; 0 bis 100 %). Fig. 2 zeigt die chromatographische Analyse von gereinigtem p-t-PA aus E. coli mittels RP HPLC. Auch bei dieser Analyse ergibt sich eine Homogenität von > 95%.

- N-terminale Aminosäuresequenz

Die N-terminale Aminosäuresequenz wurde mittels ABI 470 Sequenator mit Standardprogramm und on line PTH Detektion bestimmt. Die gefundene Sequenz S1-Y2-Q3-V4-I5 stimmt mit der aus der DNA-Sequenz abgeleiteten, zu erwartenden Aminosäuresequenz überein.

Aktivitätsbestimmung

- in vitro-Aktivität

Die in vitro-Aktivität von p-t-PA aus E. coli wurde nach der Testvorschrift für t-PA-Standard der Firma Boehringer Mannheim, Best. Nr. 1080954 bestimmt. Die spezifische Aktivität betrug 0,78 ± 0,2 x $10^6$ IU/mg. Die Stimulierbarkeit der Aktivität in diesem Testsystem durch CNBr-Fragmente des Fibrinogens (Aktivität in Gegenwart von Fibrinogenpeptiden geteilt war durch Aktivität ohne Fibrinogenpeptide) war > 10.

- in vitro-Bindung an Fibrin

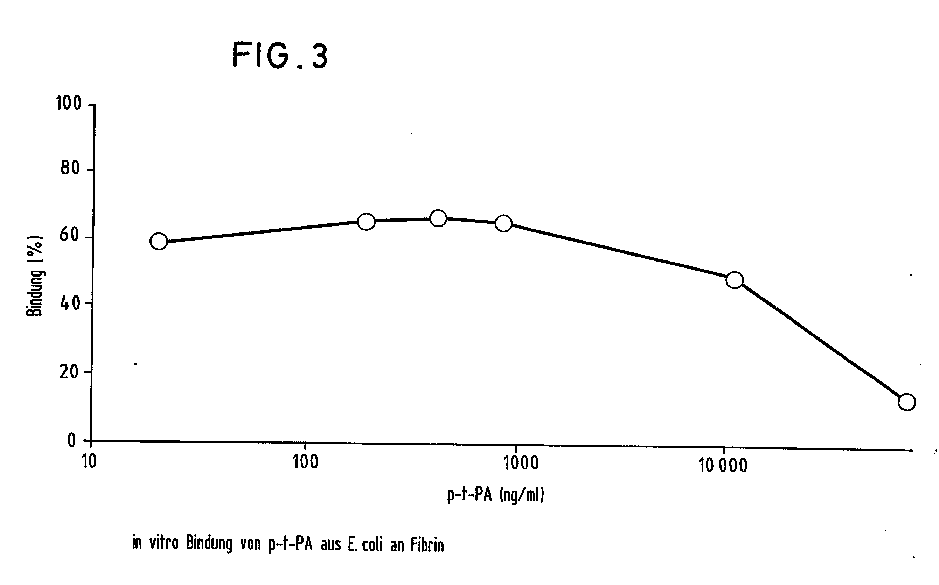

Die in vitro-Bindung von p-t-PA aus E. coli an Fibrin wurde nach der von Higgins und Vehar beschriebenen Methode bestimmt (D.L. Higgins, G.A. Vehar, Biochem. 26, (1987) 7786-91). Fig. 3 zeigt die Bindung von p-t-PA aus E. coli an Fibrin bei konstarter Fibrinkonzentration als Funktion der p-t-PA-Konzentration.

- in vivo-Thrombolyse

Die thrombolytische Wirkung von p-t-PA aus E. coli wurde am Beispiel des Jugularvenenthrombosemodells am Kaninchen in der von Collen et al. beschriebenen Form bestimmt (D. Collen, J.M. Stassen und M. Verstraete, J. Clin. Invest. 71, (1988) 368-376).

Bei einer Dosierung von 800 000 IU/kg Körpergewicht wurde eine Thrombolyse von 79 % (n = 6) erzielt. Die spontane Thrombolyse beträgt 10,8 % (n = 7), während die bei dem verwendeten Modell maximal erreichbare Thrombolyse ca. 80% betrug.

- in vivo Clearance

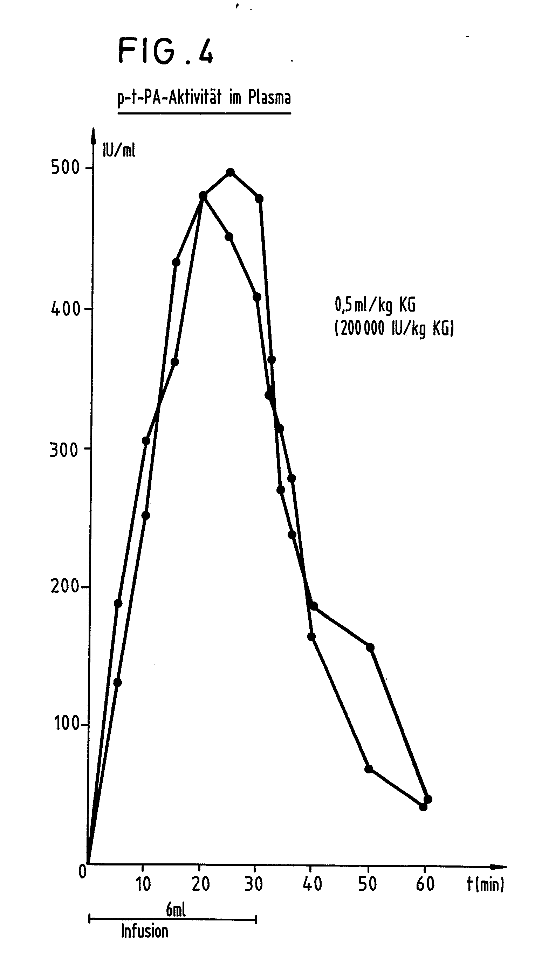

Die in vivo Clearance (Abbaurate) von p-t-PA aus E. coli wurde ebenfalls am Kaninchen überprüft. 200 000 IU/kg Körpergewicht in einem Gesamtvolumen von 6 ml wurden über 30 Minuten in die Ohrvene infundiert. Über einen Katheder in der Vena fermoralis wurden Plasmaproben entnommen. Die p-t-PA-Aktivität in den Proben wurde nach Euglobulinfällung entsprechend der Testvorschrift für t-PA-Standard (Boehringer Mannheim) bestimmt. Fig. 4 zeigt den Verlauf des Aktivitätsspiegels während und nach der Infusion.

**Beispiel 4**

Löslichkeitsverhalten von p-t-PA aus E. coli

p-t-PA aus E. coli zeigte in verdünnten wäßrigen Pufferlösungen bei physiologischen pH-Werten nur eine geringe Löslichkeit (z.B. in 0,1 mol/l Tris-HCl, pH 7,5 oder 0,1 mol/l Natriumphosphat, pH 7,5). Für eine effiziente Reinigung nach üblichen chromatographischen Trennmethoden ist es essentiell, Bedingungen zu definieren, bei denen p-t-PA aus E. coli gut löslich ist. Gleiches gilt für die Entwicklung einer pharmazeutisch verträglichen galenischen Formulierung. Im folgenden sind einige Daten zur Löslichkeit von p-t-PA aus E. coli in verschiedenen Pufferlösungen zusammengefaßt.

Löslichkeitsuntersuchungen mit konzentrierter Renaturierungslösung (vgl. Beispiel 1)

Das Konzentrat der Renaturierungslösung wurde gegen 20 mmol/l Zitronensäure, pH 2,5 bei 4°C dialysiert und zentrifugiert. Der klare Überstand wurde anschließend in 0,5 ml-Portionen bei 4°C gegen 300 ml Pufferlösung dialysiert und zentrifugiert. Die Löslichkeit wurde durch Messung der Aktivität im Überstand bestimmt.

| pH-Abhängigkeit der Löslichkeit in 20 mmol/l Zitronensäure/NaOH bzw. 20 mmol/l Natriumphosphat. | |
|---|---|
| pH | p-t-PA ($\mu$g/ml) |
| 2,5 | 403 |
| 3,5 | 420 |
| 4,5 | 38 |
| 5,5 | 5 |
| 6,5 | 3 |
| 7,5 | 2 |
| 8,5 | 4 |
| 9,5 | 6 |

| pH-Abhängigkeit der Löslichkeit in Gegenwart von 0,3 mol/l. Arginin HCl plus 20 mmol/l Zitronensäure bzw. 20 mmol/l Tris-HCl. | |
|---|---|
| pH | p-t-PA (µg/ml) |
| 2,5 | 409 |
| 3,5 | 504 |
| 4,5 | 607 |
| 5,5 | 865 |
| 6,5 | 696 |
| 7,5 | 698 |
| 8,5 | 725 |

| pH-Abhängigkeit der Löslichkeit in Gegenwart von 0,3 mol/l Guanidinium HCl plus 20 mmol/l Zitronensäure bzw. 20 mmol/l Tris-HCl. | |
|---|---|
| pH | p-t-PA (µg/ml) |
| 2,5 | 211 |
| 3,5 | 220 |
| 4,5 | 432 |
| 5,5 | 509 |
| 6,5 | 243 |
| 7,5 | 112 |
| 8,5 | 104 |

| Einfluß von Harnstoff, Harnstoffderivaten bzw. Carbonsäureamiden auf die Löslichkeit in 20 mmol/l Zitronensäure/NaOH, pH 3,5. | |
|---|---|
| Verbindung | p-t-PA ($\mu$g/ml) |
| 0,3 mol/l Harnstoff | 512 |
| 0,3 mol/l Dimethylharnstoff | 455 |
| 0,3 mol/l Tetramethylharnstoff | 539 |
| 1 mol/l Dimethylformamid | 451 |

Löslichkeitsuntersuchungen mit p-t-PA aus E. coli nach Reinigung durch Affinitätschromatographie (vgl. Beispiel 2)

Gereinigter p-t-PA aus E. coli in 0,5 mol/l L-Arginin/$H_3PO_4$, pH 7,2, 0,01 % Tween 80 wurde bei 4° C in 0,5 ml-Portionen gegen 200 ml Pufferlösung (L-Arginin/$H_3PO_4$, pH 7,2, 0,01 % Tween 80 dialysiert und zentrifugiert. Die Löslichkeit wurde durch Messung der Aktivität im Überstand bestimmt.

| L-Arginin (mol/l) | p-t-PA ($\mu$g/ml) |
|---|---|
| 0,05 | 40 |
| 0,1 | 89 |
| 0,2 | 264 |
| 0,3 | 389 |
| 0,4 | 400 |
| 0,5 | 440 |

Durch Zusatz von Tetramethylharnstoff kann die Abnahme der Löslichkeit bei niedrigen Arginin-Konzentrationen z.T. kompensiert werden.

Damit zeigt sich, daß gereinigter p-t-PA gute Löslichkeit bei einem pH von kleiner als 4, insbesondere in Gegenwart von L-Arginin oder Guanidiniumhydrochlorid besitzt.

**Ansprüche**

1. Präparat von rekombinantem, in Prokaryonten exprimiertem Plasminogenaktivator (p-t-PA), **dadurch gekennzeichnet,** daß es einen Gehalt an aktivem p-t-PA von mehr als 90%, bezogen auf Gesamtprotein, und eine spezifische Aktivität von mehr als $0,4 \times 10^6$ IU/mg aufweist.

2. Präparat nach Anspruch 1, **dadurch gekennzeichnet,** daß es einen Gehalt an aktivem p-t-PA, bezogen auf Gesamtprotein, von mehr als 95 %, insbesondere von mehr als 98 % aufweist.

3. Verfahren zur Herstellung eines Präparats von rekombinantem, in Prokaryonten exprimiertem Plasminogenaktivator (p-t-PA) mit einem Gehalt an aktivem p-t-PA von mehr als 90 %, bezogen auf Gesamtprotein, und einer spezifischen Aktivität von mehr als $0,4 \times 10^6$ IU/mg, durch Aufschluß der exprimierenden Prokaryontenzellen, Isolierung von inclusion bodies (IB's), Solubilisierung der IB's unter denaturierenden und reduzierenden Bedingungen, Derivatisierung zu gemischten Disulfiden und Reaktivierung unter kontrollierten Redox-Bedingungen, **dadurch gekennzeichnet,** daß man nach der Reaktivierung p-t-PA im Reaktivierungsansatz konzentriert und daran anschließend eine chromatographische Reinigung mittels Affinitätschromatographie durchführt.

4. Verfahren nach Anspruch 3, **dadurch gekennzeichnet,** daß man die Konzentration von p-t-PA im Reaktivierungsansatz, insbesondere für kleinere Volumina, durch eine Ammoniumsulfatfällung bewirkt.

5. Verfahren nach Anspruch 3, **dadurch gekennzeichnet,** daß man die Konzentration von p-t-PA im Reaktivierungsansatz, insbesondere bei größeren Volumina, in einem Hämodialysator durchführt.

6. Verfahren nach einem der Ansprüche 3 bis 5, **dadurch gekennzeichnet,** daß man zur chromatographischen Reinigung eine ETI-Adsorber-Säule verwendet.

7. Verfahren nach einem der Ansprüche 3 bis 6, **dadurch gekennzeichnet,** daß man in Gegenwart von 25 bis 1000 mmol/l L-Arginin arbeitet.

8. Verfahren nach einem der Ansprüche 3 bis 7, **dadurch gekennzeichnet,** daß man die chromatographische Reinigung mit dem Konzentrat des Reaktivierungsansatzes, das 25 bis 1000 mmol/l, bevorzugt 500 bis 1000 mmol/l L-Arginin enthält, über eine ETI-Adsorbersäule durchführt.

9. Verfahren nach Anspruch 8, **dadurch gekennzeichnet,** daß man das zur chromatographischen Reinigung eingesetzte Konzentrat auf einen pH-Wert von mehr als 7,0 einstellt.

10. Verfahren nach einem der Ansprüche 3 bis 9, **dadurch gekennzeichnet,** daß man die Elution bei einem pH-Wert von 5,5 bis 6,5 durchführt.

11. Verwendung eines Präparats nach Anspruch 1 oder 2 in einem Mittel zur Auflösung von Blutgerinnseln.

# FIG.1

<u>1</u>  <u>2</u>  <u>3</u>  <u>4</u>  <u>5</u>  <u>6</u>

SDS-PAGE vor und nach Reinigung mittels Affinitätschromatographie über ETI-Sepharose.
1,5:Molekulargewichtsstandard, 2,6:Renaturierungs-
lösung(Konzentrat), 3,4:p-t-PA nach Affinitätschromatographie.

# FIG.2

Absorption

Zeit
(min)

5

10

15

20

25

30 ————————————————————————30.19

35

40

RP-HPLC nach Reinigung von p-t-PA aus E.coli mittels Affinitätschromatographie über ETI-Sepharose.

# FIG. 3

in vitro Bindung von p-t-PA aus E. coli an Fibrin

EP 0 361 475 A1

FIG.4

**Europäisches Patentamt**

# EUROPÄISCHER RECHERCHENBERICHT

Nummer der Anmeldung

EP    89 11 7958

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-241022 (BOEHRINGER MANNHEIM) <br> * Seiten 15 - 18 * <br> --- | 1-3 | C07K3/08 <br> C07K3/20 <br> C12N15/58 <br> A61K37/54 |
| Y,D | EP-A-242835 (BOEHRINGER MANNHEIM) <br> * das ganze Dokument * <br> --- | 1-3 | |
| Y | EP-A-242836 (BOEHRINGER MANNHEIM) <br> * das ganze Dokument * <br> --- | 1-3 | |
| Y <br><br> D | EP-A-219874 (BOEHRINGER MANNHEIM) <br> * das ganze Dokument * <br> & EP-A-253823 <br> --- | 1-3 | |
| Y,D | EP-A-114506 (GENENTECH) <br> * Seite 48 * <br> --- | 1-3 | |
| X | MEDLINE Abstract No:83101507 <br> WALLEN P. et al: "Purification and identificatio n of two structural variant of porcine tpa..." <br> & BIOCHIM. BIOPHYS.ACTA.Nov 1982,719(2),318-28 <br> --- | 4-5 | |
| Y | EP-A-245100 (MITSUITOATSU CHEMICALS) <br> * das ganze Dokument * <br> --- | 6-10 | **RECHERCHIERTE SACHGEBIETE (Int. Cl.5)** |
| Y | EP-A-210870 (MITSUITOATSU CHEMICALS) <br> * das ganze Dokument * <br> --- | 6-10 | C12N <br> C07K <br> A61K |
| A | EP-A-217379 (MOCHIDA PHARMACEUTICAL) <br> * das ganze Dokument * <br> --- | 1-11 | |
| A | EP-A-236209 (GENETICA) <br> * Seite 21 * <br> ---- | 1-11 | |

Der vorliegende Recherchenbericht wurde für alle Patentansprüche erstellt

| Recherchenort | Abschlußdatum der Recherche | Prüfer |
|---|---|---|
| BERLIN | 04 DECEMBER 1989 | AVEDEKIAN P.F. |